# EUROPEAN PATENT SPECIFICATION

(11) **EP 1 167 514 B1**
(45) Date of publication and mention of the grant of the patent: **19.03.2003**
(21) Application number: 00830431.3
(22) Date of filing: 19.06.2000
(51) Int. Cl.: C12M 1/26

(54) **Semiautomatic apparatus for re-hydration of yeasts and process for re-hydration thereof**
Verfahren und Vorrichtung für die Rehydrierung von Hefe
Procédé et appareil semi-automatique pour la réhydratation de levures

(43) Date of publication of application: 02.01.2002
(73) Proprietor: Enologica Vason S.r.l., 37020 Pedemonte, Verona (IT)
(72) Inventor: Ferrarini, Roberto, 37022 Fumane, Verona (IT)
(74) Representative: Gerbino, Angelo

(56) References cited:
- EP-A- 0 033 987
- EP-A- 0 647 708
- EP-A- 0 794 248
- DE-A- 2 205 283
- FR-A- 2 710 073

## Description

### Field of the Invention

The present invention relates to a semiautomatic apparatus for rehydrating yeasts, and a process for the rehydration thereof.

The apparatus according to the present invention is suitable for being advantageously employed in the wine industry field in fermentation processes for the rehydration of yeasts that must be added to the medium to be fermented.

### Background of the invention

As known, for reasons of preservation and ease of transport, yeasts employed in the wine industry are generally available in their dry form.

In fact, traditionally the yeasts production process essentially involves a cell proliferation step, a subsequent drying step, and eventually a generally vacuum packaging step.

Therefore the employment of dry yeasts or of yeasts in the form of pastes in wine-making processes involves a "revitalisation" step of yeasts that must take place in the least traumatic possible way in order not to jeopardise the activity potential of the yeasts once they have been added to the must, which have already partly been attached on account of the stress they have been subjected to during dehydration.

Therefore, yeast revitalisation must be carried out keeping account of numerous parameters, such as water temperature, rehydration times, mechanical stresses, the amount of sugars and nutritional substances in general to be added, the pH, and osmotic pressure.

FR-A-2 710 073 and EP-A-0 647 708 are prior art to the present invention. More in detail the document FR-A-2 710 073 discloses an apparatus for the rehydration of dried powder of microorganisms (bacteria) for use in the treatment of effluent, which comprises a tank of freeze-dried powder and a reactor which makes it possible to revive said bacteria in a predetermined volume of liquid adjusted to an optimum temperature. The freeze-dried powder is also treated by means of a gaseous flow which provides for oxygenating and for mixing the said bacteria inside the liquid contained inside the reactor.

EP-A-0 647 708 discloses a process for the multiplication of yeast, on particular brewer's yeast, in which, in a tank-like assimilator, an amount of yeast is mixed with a nutrient solution, in particular beer wort serving as a yeast food, to give a suspension which is enriched with dissolved oxygen and to which new wort is fed stepwise and which multiplies in the environment of the tank at a controlled temperature up to a preset population density.

Currently, on the basis of the background art known to date, the process carried out for yeast rehydration in the wine industry field simply involves the execution of a number of operations in a totally manual fashion, which substantially consist of the addition of a measured dose of yeast into a vessel filled with a water content kept at a suitable temperature (generally approximately 40° C), of the subsequent shaking of the liquid in order to accomplish a distribution of the yeast, and of a waiting time for a rehydration period before adding the liquid with the rehydrated yeast into the vats containing the must to be fermented.

As it is known the indications relating to this process are generally described in detail on the yeast preservation packagings themselves and they can bear small changes such as the adding, to be carried out at the beginning, of a certain amount of sugars that are necessary in order to get yeasts to grow.

Moreover in practice, such known yeast manual rehydration process is affected by a number of both operating and functional drawbacks and shortcomings.

A first shortcoming of said process for the rehydration of yeasts is to be found in the manuality of the operations that are carried out before adding the water and rehydrated yeast solution into the must. In fact an apparatus allowing to carry out such manual operations either fully or partially automatically is not currently available.

Similar operations may be difficult to carry out especially in case the amount of product to be rehydrated is high and they may require operators having a certain expertise in order not to subject the yeasts to be rehydrated to such shocks that decrease their efficacy once they are added to the must.

Currently therefore, the yeast rehydration process results to be operatingly slightly practical and not always capable of guaranteeing a qualitatively satisfactory result (in terms of efficacy of the rehydrated yeasts) that of couse depends on the skills of the operator.

Obviously, all the manual operations of the rehydration process also require long execution times that negatively affect the overall yield of the whole wine-making production process.

Attention is also drawn to the fact that yeasts must be rehydrated during vintage, that is during a time of the year when wine producers are already strained by a considerable work overload.

A further shortcoming of the manual process for the rehydration of yeasts of the known type is the impossibility to control the parameters that concur to the rehydration and more in general to the revitalisation of the yeasts themselves.

In fact, according to the known art sugars are added to the rehydration liquid, generally water, only once and at the beginning.

Such a circumstance is an evident limitation to positive outcomes of the rehydration process, as it does not make it possible for yeasts to rehydrate and grow in optimal conditions. More in detail in fact, an optimal rehydration of yeasts requires the keeping of sugars dissolved in water at a low concentration as an excessive glucose concentration leads to the inhibition of the respiration of yeasts, thereby limiting the production of the substances that are necessary for cell functionality.

Moreover, the rehydration processes known in the art do not allow to control either of the water temperature or of the amount of oxygen dissolved in it, that are on the other hand primary decisive parameters for an optimal rehydration of yeasts.

### Summary of the Invention

A primary object of the present invention is therefore to obviate the drawbacks and shortcomings of the manual technique outlined above by providing an apparatus and a process that allow to carry out yeast rehydration in a cost-effective way whilst guaranteeing a high, constant and consistent rehydration qualitative standard.

A further object of the present invention is to provide an apparatus and a process that allow to control the parameters that concur in the determination of the efficacy of yeasts once they have been rehydrated.

A further object of the present invention is to provide an apparatus that is structurally simple and operatingly fully reliable.

The above and other objects are all achieved by the apparatus for the rehydration of yeasts according to the present invention, which comprises: a containment tank suitable for receiving a measured dose of yeasts to be rehydrated, hydraulically connected with a feeding source of a rehydration liquid, insertion means for the addition of nutritional substances inside the tank for yeast growth, a programmable logic control unit that is electrically connected with the feeding source, and with the insertion means suitable for adding the rehydration liquid and the nutritional substances to the containment tank according to predetermined sequences, whilst keeping the concentration of the nutritional substances in the rehydration liquid within a range of predetermined values.

Advantageously, the apparatus according to the invention further comprises circulation means, heating means and blowing means of a gaseous fluid respectively for mixing and heating the liquid and for dissolving a gas therein.

On account of the above apparatus and process costs involved in rehydrating yeasts are remarkably reduced, and the manual intervention of experienced operators in the rehydration process is consequently limited.

Furthermore, such an apparatus is constructively simple whilst operatingly fully realiable.

### Brief Description of the Drawings

The technical characteristics of the present invention, according to the above objects and aims, are clearly defined by the content of the appened claims and the advantages obtained therefrom are made more evident by the detailed description that follows, with reference to the attached drawing, that purely aims at representing an example thereof, where:

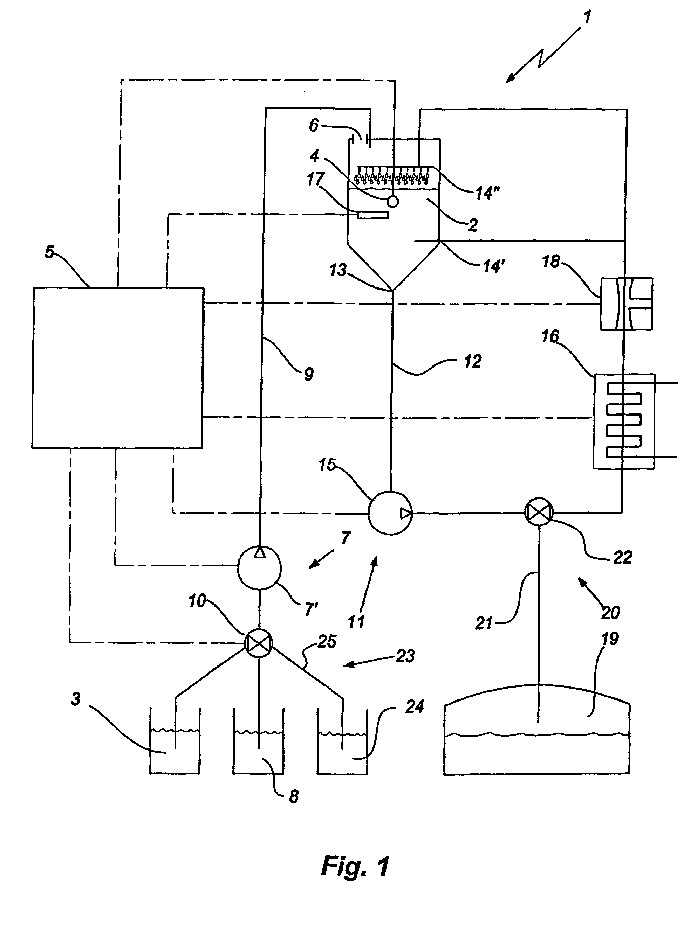

Fig. 1 illustrates a logical scheme of the operation of the apparatus for the rehydration of yeasts according to the present invention.

### Detailed Description of a Preferred Embodiment

With reference to the attached drawing the apparatus for the rehydration of yeasts is indicated with the reference numeral 1.

As it is known in the wine production field dry or paste like yeasts are usually employed in wine making processes that must be subjected to a rehydration process before being added to the must to be fermented, or to the wine if this is to be subjected to a further fermentation process.

Apparatus 1 according to the invention is capable of carrying out said rehydration in a semiautomatic fashion and for that purpose it comprises a containment tank 2 which is hydraulically connected to a feeding source 3 of a rehydration liquid that preferably consists of water. The correct amount of liquid to be added inside tank 2 is controlled by the presence of detection means 4 of the liquid level, which may advantageously be constituted by a buoy which is suitable to control the immision of water into tank 2 under the control of a programmable logic unit 5 which is more clearly described in detail hereinbelow.

The tank 2 is therefore suitable to receive a measured dose of dry yeasts by means for example of a manual addition through an upper opening 6 of tank 2.

According to the present invention, insertion means 7 for adding nutritional substances into tank 2 for yeast growth are provided. Such substances conventionally comprise concentrated must, that is a must with a high sugar content obtained by partially eliminating water, which is kept in a suitable first vessel 8 into which the insertion means 7 are dipped.

Attention is drawn however that hereafter the term nutritional substances is meant to comprise both sugars, oxygen and other edaphic or stimulating substances or fermentation regulators.

Fermentation regulators advantageously consist of a first peristaltic pump 7' or of another pumping system that is capable of dispensing fluid in a measured fashion, which is hydraulically connected to tank 2 by a first conduit 9.

According to the embodiment illustrated in the attached drawing, both the water feeding source 3, and the first vessel of concentrated must 8 (MCR) are connected to the same first pump 7' by means of a first multi-way valve 10 selectively operated on the several ports of logic control unit 5.

Therefore this logic control unit 5 is electrically connected with the feeding source 3 and with the insertion means 7 in order to control the adding of the rehydration liquid and/or of the nutritional substances to tank 2 according to predetermined operating sequences.

According to the invention, the adding of nutritional substances (i.e. of the MCR) in the rehydration liquid is carried out keeping the sugar concentration contained in the nutritional substances themselves within a range of predetermined values. The consumption of sugars by the microorganisms during the rehydration process is known per se, and in any case it can be experimentally calculated.

Excessive sugar concentrations such as those involved in processes wherein MCR is added just in the form of one solution at the beginning of the rehydration process involve a deterioration of the efficacy of the yeasts once they have been rehydrated. More particularly in fact, sugar concentrations that are not suitable, do not allow the microorganisms making up the yeasts to produce substances fulfilling a protective function against toxic agents such as alcohols during the rehydration process. Said protecting agents would otherwise allow a better efficacy of the yeasts once they have been added to the must.

For the sake of descriptive simplicity, hereafter the solution consisting of the rehydration liquid, yeasts and nutritional substances contained in tank 2 will be simply referred to as "liquid solution".

Advantageously, said apparatus 1 may further comprise circulation means 11 for mixing the liquid solution contained by tank 2. With reference to the attached drawing, these circulation means 11 are constituted by a second conduit 12 connected between an outlet opening 13 with one or more return openings 14 of the tank 2 and operatingly associated with a second pump 15 preferably a centrifuge type.

Heating means 16 may be further provided, actuable according to operating sequences programmed by the logic control unit 5 as a function of the temperature measured by suitable temperature probing means 17 of the rehydration liquid, said means being mounted on tank 2 and themselves connected with logic control unit 5.

Logic control unit 5 may be further capable of controlling the actuation of blowing means 18 of a gaseous fluid, preferably air or oxygen, into the rehydration liquid according to predetermined operating stages.

Advantageously both the heating means 16 and the blowing means 18 may be operatingly associated with the second conduit 12.

More particularly, the heating means may be constituted by a heat exchanger 16 mounted on a stretch of second conduit 12 whereas the blowing means 18 may be obtained by means of an ejector that is suitable for blowing air into the second conduit 12 by means of a venturi tube.

The second conduit 12 injects the liquid into the tank 2 by means of two openings 14, of which first opening 14' is provided on the tank 2 underneath the liquid level and second opening 14" is provided on the tank 2 above the liquid level. The return inlet opening 14" provides a rain-type addition into tank the 2 and in so doing it curbs froth development inside it that could otherwise lead to a liquid overflow out of tank 2.

Once the rehydration process has been completed, the liquid together with the rehydrated yeasts are sent to must and/or wine containment vats 19 by discharging means 20 connected hydraulically to the tank 2.

According to one preferred embodiment of the present invention, the discharging means 20 comprise a discharging conduit 21 connected with the second conduit 12 by means of a second multi-way valve 22.

Obviously, the above-described apparatus is advantageously suitable for the rehydration and proliferation not only of dry yeasts but also of paste-like yeasts, as well as of yeasts in a liquid medium or having another different form along with the rehydration and/or proliferation of other microorganisms, for example lactic bacteria.

Advantageously, once the rehydration cycle has been completed, the apparatus 1 may be subjected to a cleaning procedure that consists of circulating a detergent fluid inside the apparatus 1 by the actuation of sanitisation means 23.

More in detail, sanitisation means 23 controlled by unit 5 may be provided with a second detergent liquid vessel 24, connected with the first pump 7' by means of the first multi-way valve 10 and through a third conduit 25.

The operation of apparatus 1 according to the present invention, which has been so far described in mainly structural terms, may be better understood by means of the rehydration process according to the present invention which is described in detail below.

It essentially comprises a first injection step of a rehydration liquid inside the containment tank 2 controlled by unit 5 by means of the level detection means 4.

Thereafter there follows a first step of injection of a first measured dose of nutritional substances, among which MCR is included, for the growth of yeasts inside the tank through the actuation of first peristaltic pump 7 controlled by unit 5. After that an injection step of a measured dose of yeasts inside the tank 2 will take place for example through opening 6.

At this point a first waiting step is envisaged over a first rehydration time, normally 20 to 40 minutes, without shaking in order to let the yeasts rehydrate.

According to the invention, at the end of this stage, there follows one or more second injection stages of at least a second measured dose of nutritional substances inside the tank 2 always controlled by unit 5.

These last stages make it possible to maintain a sugar concentration falling in a range of predetermined values in the liquid solution contained in tank 2.

Straight after the first waiting stage, a mixing stage of the liquid solution contained in the tank can advantageously take place, through the actuation of the circulation means 11 controlled by unit 5, said circulation means 11 taking up the solution from the outlet opening 13 and injecting it back into the tank 2 through the return inlet openings 14' and 14".

The rehydration liquid has a temperature that is controlled in a first heating stage that takes place right after the stage wherein the liquid is injected into the tank 2.

In such a way, during the subsequent injection stage the yeasts contact the rehydration liquid at an optimal temperature and they do not run the risk of being subjected to thermal shocks. The heating stage occurs through circulation of the liquid through the second conduit 12 upon which heat exchanger 16 is mounted. When the predetermined temperature has been reached, the temperature detection means 17 by means of unit 5 control the switching off of the heating means 16.

After the first waiting stage, in case the liquid solution has a temperature that has dropped to a value that is too low, a second heating stage can take place automatically.

Following the second stage wherein a second measured dose of nutritional substances is added, a second waiting stage over a second rehydration time may take place.

Advantageously, logic control unit 5 can further control one or more injection stages of a gaseous fluid into the liquid solution contained by the tank. Such stages preferably take place after the waiting stages and during the mixing stage of the liquid solution.

More injection stages of different quantities of rehydration liquid into containment tank 2 may further be provided.

As it is known rehydrated yeasts can be employed either for fermenting must or for refermenting wine in case sparkling or fizzy wines, also known to Italians with the name of "Spumante wines", are desired or in case the exhaustion of sugars is desired.

In this latter case, when the aim is not to ferment a must devoid of alcohol but a wine with a percentage of alcohol already in it, it is preferable not to directly pour the liquid solution into the vats 19 with the wine in them, in order not to subject the rehydrated yeasts to a severe alcoholic shock, that is subsequently prone to jeopardise their efficacy. In this case, it is preferred therefore to schedule a yeasts acclimatisation stage, during which a certain amount of wine is poured into the tank 2 (for example by discharging means 20), and said amount of wine is therein kept over a time interval controlled by unit 5 before pouring the whole lot into wine the containment vats 19.

Discharging means 20 can also be employed in order to accomplish a wine and/or must circulation stage inside the tank 2, adapted to clear the tank 2 itself from the whole liquid solution contained in it.

At the end of the rehydration stage a cleaning stage of the tank 2 may follow through circulation of a detergent solution inside it. For that purpose the unit 5 actuates sanitisation means 23.

Obviously, according to the present invention all the stages described above are controlled in an automatic fashion by the logic control unit 5, which can further be programmed according to the different requirements, particularly according to the type of yeast and to the type of wine/must to be fermented.

The present invention conceived as herein detailed accomplishes the prefixed aims.

## Claims

1. Apparatus for the rehydration of yeasts, particularly in their dry or paste-like forms, comprising:
- a containment tank (2) suitable for receiving a measured dose of yeasts to be rehydrated, hydraulically connected to a feeding source (3) of a rehydration liquid,
- insertion means for the addition of nutritional substances (7) inside said containment tank (2);
- a programmable logic control unit (5) electrically connected with said feeding source (3), and with said insertion means (7) for adding said rehydration liquid and said nutritional substances inside said containment vessel (2) according to predetermined operating sequences, whilst keeping the concentration of said nutritional substances in said liquid within a range of predetermined values,
said apparatus being **characterised in that** it comprises circulation means (11) suitable for conveying the liquid solution contained inside the containment tank (2) through at least one outlet opening (13) to at least two return inlet opening (14', 14") of said containment tank (2), of which at least a first one (14') is provided on the containment tank (2) below the liquid level, and at least a second one (14") provided on the containment tank (2) above the liquid level providing a rain-like pouring into the containment tank (2) for limiting froth development in said liquid.

2. Apparatus for the rehydration of yeasts according to claim 1, **characterised in that** it comprises heating means (16) suitable for being actuated according to operating sequences programmed by said logic control unit (5) as a function of the temperature measured by temperature probing means (17) of said liquid, said temperature probing means being mounted on said containment tank (2) and themselves connected with said logic control unit (5).

3. Apparatus for the rehydration of yeasts according to claim 1, **characterised in that** it comprises blowing means (18) of a gaseous fluid into said liquid solution actuated by said logic control unit (5) according to predetermined operating stages.

4. Apparatus for the rehydration of yeasts according to claim 1, **characterised in that** said insertion means (7) for adding nutritional substances comprise a first vessel (8) containing nutritional substances connected with said containment tank (2) through a first conduit (9) operatingly associated with at least one first pump (7').

5. Apparatus for the rehydration of yeasts according to claim 1, **characterised in that** said circulation means (11) comprise a second conduit (12) connecting said outlet opening (13) with said return inlet opening (14) and operatingly associated with at least one second pump (15).

6. Apparatus for the rehydration of yeasts according to claims 2 and 5, **characterised in that** said heating means are constituted by a heat exchanger (16) associated with at least one stretch of said second conduit (12).

7. Apparatus for the rehydration of yeasts according to claims 3 and 5, **characterised in that** said blowing means (18) comprise an ejector suitable for blowing air into said second conduit (12).

8. Apparatus for the rehydration of yeasts according to claim 7, **characterised in that** said ejector blows air into said second conduit (12) by means of a venturi tube.

9. Apparatus for the rehydration of yeasts according to claim 5, **characterised in that** said second pump (15) is a centrifuge pump.

10. Apparatus for the rehydration of yeasts according to claim 5, **characterised in that** said first pump (7') is a peristaltic pump

11. Apparatus for the rehydration of yeasts according to claim 1, **characterised in that** it comprises means (4) for the detection of the liquid level that are electrically connected with said logic control unit (5).

12. Apparatus for the rehydration of yeasts according to claim 1, **characterised in that** it comprises discharging means (20) hydraulically connected to said containment tank (2) for sending the liquid solution contained therein to must/wine containment vats (19).

13. Apparatus for the rehydration of yeasts according to claims 4 and 12, **characterised in that** said discharging means (20) comprise a discharging conduit (21) connected with said second conduit (12) by means of a second multi-way valve (22).

14. Apparatus for the rehydration of yeasts according to claim 1, **characterised in that** it comprises a second vessel (24) containing detergent fluid that is suitable for circulating inside said apparatus by means of the actuation of sanitisation means (23) controlled by said logic control unit (5).

15. Apparatus for the rehydration of yeasts according to claims 4 and 8, **characterised in that** on said first conduit (9) there is mounted a first multi-way valve (10), which is connected to said first pump (7') at the outlet and to said rehydration liquid source (3) at the inlet, and further to said first and second vessels (8, 24) for selectively sending said rehydration liquid, said nutritional substances, and said detergent liquid to said containment tank (2).

16. Apparatus for the rehydration of yeasts according to claims 14 and 15, **characterised in that** said sanitisation means (23) are substantially obtained by means of said first pump (7') connected with said second vessel (24) through said first multi-way valve (10) and through a third conduit (25).

17. Process for the rehydration of yeasts in an apparatus according to any one of the preceding claims, particularly of yeasts in their dry or paste like forms comprising:
- a first stage of pouring a certain amount of rehydration liquid into a containment tank (2);
- a stage of insertion of a measured dose of yeasts is into said containment tank (2);
- a first stage of insertion of a first measured dose of nutritional substances for growth of said yeasts into said containment tank (2);
- a first waiting stage without shaking over a first rehydration time for growth and rehydration of said yeasts and;
- a second insertion stage of at least a second measured dose of nutritional substances into said containment tank (2), following said first waiting stage, in order to keep said nutritional substances in the liquid solution of said containment tank (2) in a range of predetermined values.

18. Process for the rehydration of yeasts according to claim 17, **characterised in that** it comprises a mixing stage of the liquid solution contained by the containment tank (2) by means of circulartion means (11) that are suitable for circulating said liquid solution from at least one outlet opening (13) to at least one return inlet opening (14', 14") of said containment tank (2).

19. Process for the rehydration of yeasts according to claim 17, **characterised in that** it comprises at least one first stage of heating of the liquid, prior to said insertion stage of said yeasts in said containment tank (2).

20. Process for the rehydration of yeasts according to claim 17, **characterised in that** it comprises at least one second heating stage of the liquid solution contained in the containment tank (2).

21. Process for the rehydration of yeasts according to 17, **characterised in that** it comprises at least one insertion stage of a gaseous fluid in the liquid solution contained in the containment tank (2).

22. Process for the rehydration of yeasts according to claim 17, **characterised in that** it comprises a second waiting stage over a second rehydration time, following said second insertion stage of a second measured dose of nutritional substances.

23. Process for the rehydration of yeasts according to claim 17, **characterised in that** it comprises at least one second insertion stage of a second amount of rehydration liquid into the containment tank (2) following said first waiting stage.

24. Process for the rehydration of yeasts according to claim 17, **characterised in that** it comprises at least one acclimatising stage wherein a certain amount of must/wine is inserted into said containment tank (2) and said amount of must/wine is kept therein over a predetermined time lapse prior to a subsequent reinsertion of the full amount of product contained in the containment tank (2) into containment vats (19) for must and/or wine.

25. Process for the rehydration of yeasts according to claim 17, **characterised in that** it comprises at least one circulation stage of must/wine inside the containment tank (2) in order to transfer all of the liquid solution therein contained into containment vats (19) for wine and/or must.

26. Process for the rehydration of yeasts according to claim 17, **characterised in that** it comprises at least one cleaning stage of the containment tank (2) by means of circulating a detergent solution inside it.

27. Process for the rehydration of yeasts according to anyone of claims 17 and 26, **characterised in that** each of the said stages is controlled by a programmable logic control unit (5).

## Patentansprüche

1. Vorrichtung zur Rehydratisierung von Hefen, insbesondere in deren trockenen oder pastenartigen Formen, welche umfasst:
- einen zum Aufnehmen einer bemessenen Dosis von zu rehydratisierenden Hefen geeigneten Einschlussbehälter (2), welcher mit einer Quelle (3) zum Versorgen mit einer Rehydratisierungsflüssigkeit hydraulisch verbunden ist,
- Einbringmittel für die Zugabe von Nährsubstanzen (7) innerhalb des Einschlussbehälters (2);
- eine programmierbare logische Steuereinheit (5), welche elektrisch verbunden ist mit der Versorgungsquelle (3) und mit dem Einbringmittel (7) zum Zugeben der Rehydratisierungsflüssigkeit und der Nährsubstanzen innerhalb des Einschlussbehälters (2) gemäß vorbestimmter Funktionsabläufe, während die Konzentration der Nährsubstanzen in der Flüssigkeit innerhalb eines Bereichs von vorbestimmten Werten gehalten wird,
welche Vorrichtung **dadurch gekennzeichnet ist, dass** sie Umwälzmittel (11) umfasst, die geeignet sind, die innerhalb des Einschlussbehälters (2) enthaltene flüssige Lösung durch wenigstens eine Auslassöffnung (13) zu wenigstens zwei Rücklaufeinlassöffnungen (14', 14") des Einschlussbehälters (2) zu befördern, von denen wenigstens eine erste (14') an dem Einschlussbehälter (2) unterhalb des Flüssigkeitspegels angeordnet ist, und wenigstens eine zweite (14"), die an dem Einschlussbehälter (2) oberhalb des Flüssigkeitspegels angeordnet ist, ein regenartiges Eingießen in den Einschlussbehälter (2) zum Begrenzen der Schaumentwicklung in der Flüssigkeit ermöglicht.

2. Vorrichtung zur Rehydratisierung von Hefen nach Anspruch 1, **dadurch gekennzeichnet, dass** sie Heizmittel (16) umfasst, die entsprechend von Funktionsabläufen, die in der logischen Steuereinheit (5) programmiert sind, abhängig von der von Temperaturerfassungsmitteln (17) der Flüssigkeit gemessenen Temperatur betätigt werden können, welche Temperaturerfassungsmittel an den Einschlussbehälter (2) montiert und ihrerseits mit der logischen Steuereinheit (5) verbunden sind.

3. Vorrichtung zur Rehydratisierung von Hefen nach Anspruch 1, **dadurch gekennzeichnet, dass** sie Mittel (18) zum Blasen eines gasförmigen Fluids in die flüssige Lösung umfasst, welche durch die logische Steuereinheit (5) gemäß vorbestimmter Funktionsschritte betätigt werden.

4. Vorrichtung zur Rehydratisierung von Hefen nach Anspruch 1, **dadurch gekennzeichnet, dass** die Einbringmittel (7) zum Zugeben von Nährsubstanzen ein Nährsubstanzen enthaltendes erstes Gefäß (8) umfassen, welches mit dem Einschlussbehälter (2) über einen ersten Gang (9) verbunden ist, der funktionell mit wenigstens einer ersten Pumpe (7') verbunden ist.

5. Vorrichtung zur Rehydratisierung von Hefen nach Anspruch 1, **dadurch gekennzeichnet, dass** die Umwälzmittel (11) einen zweiten Gang (12) umfassen, der die Auslassöffnung (13) mit der Rücklaufeinlassöffnung (14) verbindet und funktionell mit wenigstens einer zweiten Pumpe (15) verbunden ist.

6. Vorrichtung zur Rehydratisierung von Hefen nach den Ansprüchen 2 und 5, **dadurch gekennzeichnet, dass** die Heizmittel durch einen Wärmetauscher (16) gebildet sind, der mit wenigstens einer Ausdehnung des zweiten Gangs (12) zusammenwirkt.

7. Vorrichtung zur Rehydratisierung von Hefen nach den Ansprüchen 3 und 5, **dadurch gekennzeichnet, dass** die Blasmittel (18) einen Ejektor umfassen, der zum Blasen von Luft in den zweiten Gang (12) geeignet ist.

8. Vorrichtung zur Rehydratisierung von Hefen nach Anspruch 7, **dadurch gekennzeichnet, dass** der Ejektor mittels eines Venturi-Rohrs Luft in den zweiten Gang (12) bläst.

9. Vorrichtung zur Rehydratisierung von Hefen nach Anspruch 5, **dadurch gekennzeichnet, dass** die zweite Pumpe (15) eine Zentrifugenpumpe ist.

10. Vorrichtung zur Rehydratisierung von Hefen nach Anspruch 5, **dadurch gekennzeichnet, dass** die erste Pumpe (7') eine peristaltische Pumpe ist.

11. Vorrichtung zur Rehydratisierung von Hefen nach Anspruch 1, **dadurch gekennzeichnet, dass** sie Mittel (4) zur Erfassung des Flüssigkeitspegels umfasst, welche mit der logischen Steuereinheit (5) elektrisch verbunden sind.

12. Vorrichtung zur Rehydratisierung von Hefen nach Anspruch 1, **dadurch gekennzeichnet, dass** sie Entlademittel (20) umfasst, welche mit dem Einschlussbehälter (2) hydraulisch verbunden sind um die darin enthaltene Flüssigkeitslösung in Most-/Weinfässer (19) zu schicken.

13. Vorrichtung zur Rehydratisierung von Hefen nach den Ansprüchen 4 und 12, **dadurch gekennzeichnet, dass** die Entlademittel (20) einen Entladegang (21) umfassen, welcher mit dem zweiten Gang (12) über ein zweites Mehrwegventil (22) verbunden ist.

14. Vorrichtung zur Rehydratisierung von Hefen nach Anspruch 1, **dadurch gekennzeichnet, dass** sie ein zweites Gefäß (24) umfasst, das eine Reinigungsflüssigkeit enthält, das zum Umwälzen innerhalb der Vorrichtung mittels der Betätigung von durch die logische Steuereinheit (5) gesteuerten Hygienisierungsmitteln (23) geeignet ist.

15. Vorrichtung zur Rehydratisierung von Hefen nach den Ansprüchen 4 und 8, **dadurch gekennzeichnet, dass** an dem ersten Gang (9) ein erstes Mehrwegventil (10) montiert ist, das mit der ersten Pumpe (7') am Auslass und mit der Rehydratisierungsflüssigkeitsquelle (3) am Einlass, und ferner mit den ersten und zweiten Gefäßen (8, 24) verbunden ist, um die Rehydratisierungsflüssigkeit, die Nährsubstanzen und die Reinigungsflüssigkeit selektiv zu dem Einschlussbehälter (2) zu schicken.

16. Vorrichtung zur Rehydratisierung von Hefen nach den Ansprüchen 14 und 15, **dadurch gekennzeichnet, dass** die Hygienisierungsmittel (23) im wesentlichen durch die erste Pumpe (7') erhalten werden, die durch das erste Mehrwegventil (10) und durch einen dritten Gang (25) mit dem zweiten Gefäß (24) verbunden ist.

17. Verfahren zur Rehydratisierung von Hefen in einer Vorrichtung nach einem der vorhergehenden Ansprüche, insbesondere von Hefen in ihren trockenen oder pastenartigen Formen, welches umfasst:
- ein erster Schritt zum Gießen einer bestimmten Menge von Rehydratisierungsflüssigkeit in einen Einschlussbehälter (2);
- ein Schritt zum Einbringen einer bemessenen Dosis von Hefen in den Einschlussbehälter (2);
- ein erster Schritt zum Einbringen einer ersten bemessenen Dosis von Nährsubstanzen zum Wachstum der Hefen in den Einschlussbehälter (2);
- ein erster Schritt zum Warten ohne Schütteln für eine erste Rehydratisierungszeit zum Wachstum und Rehydratisieren der Hefen und;
- ein zweiter Schritt zum Einbringen von wenigstens einer zweiten bemessenen Dosis von Nährsubstanzen in den Einschlussbehälter (2), welcher dem ersten Warteschritt folgt, um die Nährsubstanzen in der Flüssigkeitslösung des Einschlussbehälters (2) in einem Bereich von vorbestimmten Werten zu halten.

18. Verfahren zur Rehydratisierung von Hefen nach Anspruch 17, **dadurch gekennzeichnet, dass** es einen Schritt zum Mischen der in dem Einschlussbehälter (2) enthaltenen flüssigen Lösung mittels Umwälzmittel (11), welche zum Umwälzen der flüssigen Lösung von wenigstens einer Auslassöffnung (13) zu wenigstens einer Rückkehreinlassöffnung (14', 14") des Einschlussbehälters (2) geeignet sind, umfasst.

19. Verfahren zur Rehydratisierung von Hefen nach Anspruch 17, **dadurch gekennzeichnet, dass** es wenigstens einen ersten Schritt zum Heizen der Flüssigkeit vor dem Schritt zum Einbringen der Hefen in den Einschlussbehälter (2) umfasst.

20. Verfahren zur Rehydratisierung von Hefen nach Anspruch 17, **dadurch gekennzeichnet, dass** es wenigstens einen zweiten Schritt zum Heizen der in dem Einschlussbehälter (2) enthaltenen flüssigen Lösung umfasst.

21. Verfahren zur Rehydratisierung von Hefen nach Anspruch 17, **dadurch gekennzeichnet, dass** es wenigstens einen Schritt zum Einbringen eines gasförmigen Fluids in die in dem Einschlussbehälter (2) enthaltene flüssige Lösung umfasst.

22. Verfahren zur Rehydratisierung von Hefen nach Anspruch 17, **dadurch gekennzeichnet, dass** es einen zweiten Schritt zum Warten für eine zweite Rehydratisierungszeit, welcher dem zweiten Schritt zum Einfügen einer zweiten bemessenen Dosis von Nährsubstanzen folgt, umfasst.

23. Verfahren zur Rehydratisierung von Hefen nach Anspruch 17, **dadurch gekennzeichnet, dass** es einen zweiten Schritt zum Einbringen einer zweiten Menge von Rehydratisierungsflüssigkeit in den Einschlussbehälter (2), welcher dem ersten Warteschritt folgt, umfasst.

24. Verfahren zur Rehydratisierung von Hefen nach Anspruch 17, **dadurch gekennzeichnet, dass** es wenigstens einen Akklimatisierungsschritt umfasst, bei welchem eine bestimmte Menge von Most/Wein in den Einschlussbehälter (2) eingebracht wird und die Menge von Most/Wein über einen vorbestimmten Zeitraum darin gehalten wird, vor einem darauffolgenden Wiedereinbringen der vollen Menge des in dem Einschlussbehälters (2) enthaltenen Produkts in Einschlussfasser (19) für Most und/oder Wein.

25. Verfahren zur Rehydratisierung von Hefen nach Anspruch 17, **dadurch gekennzeichnet, dass** es wenigstens einen Schritt zum Umwälzen von Most/Wein innerhalb des Einschlussbehälters (2) umfasst, um die gesamte darin enthaltene flüssige Lösung in Einschlussfässer (19) für Wein und/oder Most zu überführen.

26. Verfahren zur Rehydratisierung von Hefen nach Anspruch 17, **dadurch gekennzeichnet, dass** es wenigstens einen Schritt zum Reinigen des Einschlussbehälters (2) mittels Umwälzen einer darin befindlichen Reinigungslösung umfasst.

27. Verfahren zur Rehydratisierung von Hefen nach den Ansprüchen 17 und 26, **dadurch gekennzeichnet, dass** jeder der Schritte von einer programmierbaren logischen Steuereinheit (5) gesteuert wird.

## Revendications

1. Dispositif destiné à la réhydratation des levures, en particulier dans leur forme sèche ou dans leur forme semblable à de la pâte, comprenant :
un réservoir de rétention (2) adapté pour recevoir une dose mesurée de levures devant être réhydratées, relié hydrauliquement à une source d'alimentation (3) d'un liquide de réhydratation,
des moyens d'introduction pour ajouter des substances nutritionnelles (7) à l'intérieur dudit réservoir de rétention (2) ;
une unité de commande logique programmable (5) reliée électriquement à ladite source d'alimentation (3), et auxdits moyens d'introduction (7) pour ajouter ledit liquide de réhydratation et lesdites substances nutritionnelles à l'intérieur dudit réservoir de rétention (2) selon des phases de travail prédéterminées, en maintenant la concentration desdites substances nutritionnelles dans ledit liquide dans un intervalle de valeurs prédéterminées,
ledit dispositif étant **caractérisé en ce qu'**il comprend un moyen de circulation (11) adapté pour transporter la solution liquide contenue à l'intérieur du réservoir de rétention (2) à travers au moins une ouverture de sortie (13) vers au moins deux ouvertures d'admission (14', 14'') des retours de la solution liquide dudit réservoir de rétention (2), dont au moins une première ouverture (14') est proposée sur le réservoir de rétention (2) en dessous du niveau du liquide, et au moins une deuxième ouverture (14'') est proposée sur le réservoir de rétention (2) au-dessus du niveau du liquide proposant un versement en pluie dans le réservoir de rétention (2) pour limiter le développement de mousse dans ledit liquide.

2. Dispositif destiné à la réhydratation des levures selon la revendication 1, **caractérisé en ce qu'**il comprend des moyens de chauffage (16) adaptés pour être actionnés selon des phases de travail programmées par ladite unité de commande logique (5) comme une fonction de la température mesurée par les moyens de sondage de la température (17) dudit liquide, lesdits moyens de sondage de la température étant montés sur ledit réservoir de rétention (2) et eux-mêmes reliés à ladite unité de commande logique (5).

3. Dispositif destiné à la réhydratation des levures selon la revendication 1, **caractérisé en ce qu'**il comprend des moyens de soufflage (18) d'un fluide gazeux dans ladite solution liquide actionnés par ladite unité de commande logique (5) selon des phases de travail prédéterminées.

4. Dispositif destiné à la réhydratation des levures selon la revendication 1, **caractérisé en ce que** lesdits moyens d'introduction (7) pour ajouter des substances nutritionnelles comprennent un premier réservoir (8) contenant des substances nutritionnelles, relié audit réservoir de rétention (2) par un premier conduit (9) associé en fonctionnement à au moins une première pompe (7').

5. Dispositif destiné à la réhydratation des levures selon la revendication 1, **caractérisé en ce que** ledit moyen de circulation (11) comprend un deuxième conduit (12) reliant ladite ouverture de sortie (13) à ladite ouverture d'admission (14) et associé en fonctionnement à au moins une deuxième pompe (15).

6. Dispositif destiné à la réhydratation des levures selon les revendications 2 et 5, **caractérisé en ce que** lesdits moyens de chauffage sont constitués par un échangeur de chaleur (16) associé à au moins une extension dudit deuxième conduit (12).

7. Dispositif destiné à la réhydratation des levures selon les revendications 3 à 5, **caractérisé en ce que** les moyens de soufflage (18) comprennent un éjecteur adapté pour souffler de l'air dans ledit deuxième conduit (12).

8. Dispositif destiné à la réhydratation des levures selon la revendication 7, **caractérisé en ce que** ledit éjecteur souffle de l'air dans ledit deuxième conduit (12) au moyen d'un tube Venturi.

9. Dispositif destiné à la réhydratation des levures selon la revendication 5, **caractérisé en ce que** ladite deuxième pompe (15) est une pompe centrifuge.

10. Dispositif destiné à la réhydratation des levures selon la revendication 5, **caractérisé en ce que** ladite deuxième pompe (7') est une pompe péristaltique.

11. Dispositif destiné à la réhydratation des levures selon la revendication 1, **caractérisé en ce qu'**il comprend des moyens (4) pour la détection du niveau de liquide qui sont reliés électriquement à ladite unité de commande logique (5).

12. Dispositif destiné à la réhydratation des levures selon la revendication 1, **caractérisé en ce qu'**il comprend des moyens de déchargement (20) reliés hydrauliquement audit réservoir de rétention (2) pour envoyer la solution liquide contenue dans ledit réservoir de rétention vers des cuves de rétention (19) de moût/vin.

13. Dispositif destiné à la réhydratation des levures selon les revendications 4 et 12, **caractérisé en ce que** lesdits moyens de déchargement (20) comprennent un conduit de déchargement (21) relié audit deuxième conduit (12) au moyen d'une deuxième vanne à voies multiples (22).

14. Dispositif destiné à la réhydratation des levures selon la revendication 1, **caractérisé en ce qu'**il comprend un deuxième réservoir (24) contenant un liquide détergent qui est adapté pour circuler à l'intérieur dudit dispositif au moyen de l'actionnement des moyens de nettoyage (23) commandés par ladite unité de commande logique (5).

15. Dispositif destiné à la réhydratation des levures selon les revendications 4 et 8, **caractérisé en ce que** sur ledit premier conduit (9), une première vanne à voies multiples (10) est montée, qui est reliée à ladite première pompe (7') à la sortie et à ladite source (3) du liquide de réhydratation à l'admission, et en plus auxdits premier et deuxième réservoirs (8, 24) pour envoyer de manière sélective ledit liquide de réhydratation, lesdites substances nutritionnelles, et ledit liquide détergent audit réservoir de rétention (2).

16. Dispositif destiné à la réhydratation des levures selon les revendications 14 et 15, **caractérisé en ce que** lesdits moyens de nettoyage (23) sont sensiblement obtenus au moyen de ladite première pompe (7') reliée audit deuxième réservoir (24) à travers ladite première vanne à voies multiples (10) et à travers ledit troisième conduit (25).

17. Procédé destiné à la réhydratation des levures dans un dispositif selon l'une quelconque des revendications précédentes, en particulier des levures dans leur forme sèche ou dans leur forme semblable à de la pâte comprenant :
une première étape consistant à verser une certaine quantité de liquide de réhydratation dans un réservoir de rétention (2) ;
une étape d'introduction d'une dose mesurée de levures a lieu dans ledit réservoir de rétention (2) ;
une première étape d'introduction d'une première dose mesurée de substances nutritionnelles pour la croissance desdites levures dans ledit réservoir de rétention (2) ;
une première étape d'attente sans secouer au premier moment de réhydratation pour la croissance et la réhydratation desdites levures et ;
une deuxième étape d'introduction d'au moins une deuxième dose mesurée de substances nutritionnelles dans ledit réservoir de rétention (2), à la suite de ladite première étape d'attente, afin de maintenir lesdites substances nutritionnelles dans la solution liquide dudit réservoir de rétention (2) dans un intervalle de valeurs prédéterminées.

18. Procédé destiné à la réhydratation des levures selon la revendication 17, **caractérisé en ce qu'**il comprend une étape de mélange de la solution liquide contenue par le réservoir de rétention (2) à l'aide des moyens de circulation (11) qui sont adaptés pour faire circuler ladite solution liquide d'au moins une ouverture de sortie (13) vers au moins une ouverture d'admission (14', 14'') dudit réservoir de rétention (2).

19. Procédé destiné à la réhydratation des levures selon la revendication 17, **caractérisé en ce qu'**il comprend au moins une première étape de chauffage du liquide, avant ladite étape d'introduction desdites levures dans ledit réservoir de rétention (2).

20. Procédé destiné à la réhydratation des levures selon la revendication 17, **caractérisé en ce qu'**il comprend au moins une deuxième étape de chauffage de la solution liquide contenue dans le réservoir de rétention (2).

21. Procédé destiné à la réhydratation des levures selon la revendication 17, **caractérisé en ce qu'**il comprend au moins une étape d'introduction d'un liquide gazeux dans la solution liquide contenue dans le réservoir de rétention (2).

22. Procédé destiné à la réhydratation des levures selon la revendication 17, **caractérisé en ce qu'**il comprend une deuxième étape d'attente pendant un deuxième moment de réhydratation, à la suite de ladite deuxième étape d'introduction d'une deuxième dose mesurée de substances nutritionnelles.

23. Procédé destiné à la réhydratation des levures selon la revendication 17, **caractérisé en ce qu'**il comprend au moins une deuxième étape d'introduction d'une deuxième quantité de liquide de réhydratation dans le réservoir de rétention (2) à la suite de ladite première étape d'attente.

24. Procédé destiné à la réhydratation des levures selon la revendication 17, **caractérisé en ce qu'**il comprend au moins une étape d'acclimatation dans laquelle une certaine quantité de moût/vin est introduite dans ledit réservoir de rétention (2) et **en ce que** ladite quantité de moût/vin y est conservée pendant un laps de temps prédéterminé avant une réintroduction subséquente de la quantité complète de produit contenue dans le réservoir de rétention (2) dans les cuves de rétention (19) destinées au moût et/ou au vin.

25. Procédé destiné à la réhydratation des levures selon la revendication 17, **caractérisé en ce qu'**il comprend au moins une étape de circulation du moût/vin à l'intérieur du réservoir de rétention (2) afin de transférer la totalité de la solution liquide contenue dans ledit réservoir de rétention dans les cuves de rétention (19) destinées au vin et/ou au moût.

26. Procédé destiné à la réhydratation des levures selon la revendication 17, **caractérisé en ce qu'**il comprend au moins une étape de nettoyage du réservoir de rétention (2) en y faisant circuler une solution détergente.

27. Procédé destiné à la réhydratation des levures selon les revendications 17 et 26, **caractérisé en ce que** chacune desdites étapes est commandée par une unité de commande logique programmable (5).
